# EUROPEAN PATENT APPLICATION

(11) **EP 4 248 985 A1**
(43) Date of publication of application: **27.09.2023**
(21) Application number: 22382277.6
(22) Date of filing: 25.03.2022
(51) Int. Cl.: A61K 38/10, A61P 27/02

(54) **PEPTIDES FOR THE TREATMENT OF RETINITIS PIGMENTOSA**

(71) Applicant: Consejo Superior de Investigaciones Científicas (CSIC), 28006 Madrid (ES)
(72) Inventor: HERNÁNDEZ SÁNCHEZ, Catalina, Madrid (ES); DE LA ROSA CANO, Enrique, Madrid (ES)
(74) Representative: Cueto, Sénida

(57) **Abstract**

The present invention relates to a synthetic peptide analog of the Preimplantation factor (PIF) peptide with a sequence length of maximum 20 amino acids consisting in the sequence Xaa1Xaa2R314K5P6Xaa7-20 (SEQ ID No1): wherein Xaa can be any amino acid for use in the treatment and/or prevention of Retinitis Pigmentosa and a pharmaceutical composition comprising one or more synthetic peptide analog from PIF peptides and one or more acceptable carriers.

## Description

### Field of the Invention

The present invention belongs to the field of vision health; it discloses peptides for use in the treatment of Retinitis Pigmentosa, and a pharmaceutical composition comprising one or more of said peptides.

### Background

Retinitis pigmentosa (RP) is a group of retinal genetic dystrophies responsible for the most cases of hereditary blindness. RP is considered a rare disease with a prevalence of 1/3,000-1/4,000. RP encompasses a range of genetically heterogeneous disorders caused by more than 3,000 different mutations in over 60 genes. Despite its genetic heterogeneity, most of all forms of RP include primary dysfunction and death of rod photoreceptors causing night blindness and constriction of the visual field, followed by the secondary loss of cones that eventually results in blindness. At the present, RP is an unpreventable and incurable disease. Although gene therapy will constitute the ultimate treatment, because of its complex genetic etiology gene-independent therapies may constitute a valuable initial alternative to attenuate vision loss.

The scientific article Hayrabedyan, Soren et al. "The core sequence of PIF competes for insulin/amyloid β in insulin degrading enzyme: potential treatment for Alzheimer's disease." Oncotarget vol. 9,74 33884-33895. 21 Sep. 2018, doi:10.18632/oncotarget.26057 discloses a synthetic analog PIF peptide for the potential treatment of Alzheimer's disease, which allows the proteolytic degradation of the amyloid-β peptides responsible of Alzheimer's disease. However, this article does not suggest the possibility of treatment of visual diseases such as Retinitis Pigmentosa. The accumulation of amyloid-β peptides has not been described in RP.

The scientific article Weiss, Lola et al. "Preimplantation factor (PIF*) reverses neuroinflammation while promoting neural repair in EAE model." Journal of the neurological sciences vol. 312,1-2 (2012): 146-57. doi:10.1016/j.jns.2011.07.050 discloses a synthetic analog of the PIF peptide for the reversion of the neuroinflammation. However, this document does not disclose the mechanism of action nor suggests the use of the above-mentioned peptides in RP.

The patent document EP1879604B1 discloses several PIF derived peptides with a therapeutic effect in the modulation of the immune system. However, this document does not disclose the PIF derived peptides of the invention, nor its use in the treatment of Retinitis Pigmentosa.

The patent document EP2968471B1 discloses several peptides for the treatment of retinal neurodegenerative diseases. However, it does not suggest the use of synthetic analogs of PIF derived peptides nor the delay in loss of visual function.

The present invention discloses a synthetic peptide derived from the Preimplantation factor (PIF) peptide for the therapeutic treatment of a patient suffering from Retinitis Pigmentosa resulting in a delay of the loss of visual function and preservation of photoreceptor cells.

Moreover, treatment with synthetic peptide analogs of PIF results in better preservation of the retinal structure and function in mouse models for RP. Overall, these results support the use of PIF peptides as a therapeutic approach in this disease.

### Description

The terms "Retinitis pigmentosa" and "RP" are synonyms and can be interchangeably used.

The term "patient" as used herein refers to human beings. The term "patient" is not intended to be limiting in any respect, and it may be of any age, sex and physical condition.

The expression "use in the treatment" as used herein means: preventing RP or the development of RP; inhibiting the progression of RP; arresting or preventing the further development of RP; reducing the severity of RP; ameliorating or alleviating symptoms associated with RP; and causing regression of RP or of one or more of the symptoms associated with RP.

The expression "therapeutically effective amount" as used herein, refers to the amount of a compound that, when administered, is sufficient to prevent the development of, or alleviate to some extent, one or more of the symptoms of RP. The particular dose of the compound administered according to this invention will of course be determined by the particular circumstances surrounding the case, including the compound administered, the route of administration, and similar considerations.

The term "pharmaceutically acceptable" as used herein pertains to compounds, materials, compositions, and/or dosage forms which are, within the scope of sound medical judgment, suitable for the use in contact with the tissues of a subject (e.g. human) without significant toxicity, irritation, allergic response, or other problem or complication, commensurate with a reasonable benefit/risk ratio. Each carrier, excipient, etc., must also be "acceptable" in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. Suitable carriers, excipients, etc. can be found in standard pharmaceutical texts, and include, as a way of example, preservatives, agglutinants, humectants, emollients, and antioxidants.

The terms "sPIF" and "PIF peptides" are synonyms and can be interchangeably used.

The present invention relates to biological effects induced in vitro and/or in vivo by preimplantation factor (PIF) peptides, peptidomimetics, and compounds derived or analogs thereof that harbors in part, or homologous to the amino acid sequence of PIF peptides or to the scrambled amino acid sequence of PIF peptides. In particular, until now it has not been demonstrated that a synthetic peptide comprising or mimicking the structural and biological features of the PIF peptide exerts a therapeutic effect on Retinitis Pigmentosa.

Accordingly, this synthetic peptide analog of PIF peptide and molecules that mimic the structure and function of this peptide may be used as therapeutic agents in Retinitis Pigmentosa.

The first object of the present invention relates to a synthetic peptide analog of the Preimplantation factor (PIF) peptide with a sequence length of maximum 20 amino acids consisting in the sequence:
Xaa₁ Xaa₂ R₃ I₄ K₅ P₆ Xaa₇₋₂₀ (SEQ ID No1): wherein:
Xaa can be any amino acid for use in the treatment of Retinitis Pigmentosa.

In a particular embodiment, the synthetic peptide analog of the PIF peptide has a sequence length of 10 to 20 amino acids residues, preferably of 11 to 19 amino acid residues, more preferably of 12 to 18 amino acid residues, even more preferably of 13 to 17 amino acid residues, even more preferably of 14 to 16 amino acid residues and even more preferably of 15 amino acids residues.

In another particular embodiment the synthetic peptide analog of the PIF peptide is MVRIKPGSANKPSDD (SEQ_ID No 2).

In a particular embodiment, the synthetic peptide analog of the Preimplantation factor (PIF) peptide with an identity of at least 90%, preferably 95% with respect to the SEQ_ID No 2 for use in the treatment of Retinitis Pigmentosa.

In another particular embodiment one or more amino acid residues of SEQ_ID No 2 may be replaced by another amino acid residue which does not substantially alters its biological function because they have similar biochemical properties. This is called conservative replacement. For example:
One or more aliphatic amino acids can be substituted by one or more aliphatic amino acids: Glycine, Alanine, Valine, Leucine and Isoleucine.
One or more aromatic amino acids can be substituted by one or more aromatic amino acids: Phenylalanine, Tyrosine and Tryptophan.
One or more basic amino acids can be substituted by one or more basic amino acids: Histidine, Lysine and Arginine.
One or more acidic amino acids can be substituted by one or more acidic amino acids: Aspartate, Glutamate, Asparagine, Glutamine.

In another particular embodiment, the synthetic peptide analog of the PIF peptide comprises the amino acid sequence of the PIF peptide of a mammal, for example: dog, cat, mouse, rat, rabbit, pig, monkey.

In another embodiment of the invention, one or more amino acid residues of the peptide of the invention can be chemically modified by phosphorylation, sulfonation or biotinylation, or be pegylated, or be substituted with a D amino acid.

This means that any of the peptides with any sequence length and amino acid composition as stated above may be used in the manufacture of a medicament for the treatment and/or prevention of Retinitis Pigmentosa, in particular for the treatment and/or prevention of the retinal degeneration at early stages of the disease. The treatment and/or prevention comprises administering therapeutically effective amounts of a peptide with any of the sequence lengths and amino acid compositions as stated above.

The amount of a synthetic peptide analog of PIF peptide administered to a patient may be at a dose of 0,1 mg/kg to 1000 mg/kg, preferably a dose of 0,1 mg/kg to 500 mg/kg, more preferably a dose of 0,1 mg/kg to 400 mg/kg, or even more preferably a dose of 0,1 mg/kg to 300 mg/kg.

The synthetic analog derived from PIF peptide may be administered in saline solution: NaCl 0.9% w/v.

Another object of the present invention relates to a pharmaceutical composition for use in the treatment and/or prevention of Retinitis Pigmentosa, which comprises at least one synthetic peptide analog of the Preimplantation factor (PIF) peptide as defined above, or a pharmaceutically acceptable salt of said peptide, and a pharmaceutically acceptable carrier.

In a particular embodiment of the pharmaceutical composition, the synthetic peptide analog of the PIF peptide sequence is MVRIKPGSANKPSDD.

In another embodiment, the pharmaceutical composition for use according to the invention, is an ingredient (component) of a pharmaceutical composition, said composition comprising at least one peptide as above disclosed and at least one pharmaceutically acceptable carrier and/or excipient.

Thus, the invention relates also to a pharmaceutical composition for use in the treatment and/or prevention of Retinitis Pigmentosa, which comprises at least one peptide as defined above. Particular excipients are selected from preservatives, agglutinants, humectants, emollients, antioxidants, water, saline buffers, and mixtures of water in oil or oil in water.

Additionally, the compositions of the present invention may contain other ingredients, such as fragrances, colorants, and other components known in the state of the art for their use in specific formulations.

Preferred pharmaceutical compositions are selected from the group consisting of solutions (for example eye drops), creams, lotions, unguents, emulsions, aerosols and non-aerosol sprays, gels, ointments and suspensions.

Compositions of the present invention can be prepared according to methods well known in the state of the art. The appropriate excipients and/or carriers, and their amounts, can readily be determined by those skilled in the art according to the type of formulation being prepared.

The pharmaceutical compositions of the invention can be administered to a patient topically, intranasally, orally, subcutaneously, intravitreally, subjunctivally, intramuscularly or in a form of eye drops.

In another particular embodiment, the pharmaceutical composition can be administered with a variable administration interval, by way of example from 1 to 7 times per week, preferably from 2 to 6 times per week, more preferably from 3 to 5 times per week.

In a particular embodiment, the pharmaceutical composition is administered as a delayed release composition.

The pharmaceutical composition administered intravitreally and or subjunctivally may be loaded in a drug delivery system as described in Drug Discovery Series No. 66 Therapies for Retinal Degeneration: Targeting Common Processes; Chapter 10: Drug Delivery Systems for the Treatment of Diseases Affecting the Retina and Optic Nerve; Authors: I. Bravo-Osuna, V. Andrés Guerrero, I. T. Molina-Martinez, and R. Herrero-Vandrell.

The pharmaceutical composition administered intravitreally may be loaded in a PLGA microsphere formulation (biodegradable poly (lactic-co-glycolic acid) microspheres) as described in Isiegas C, Marinich-Madzarevich JA, Marchena M, et al. Intravitreal Injection of Proinsulin-Loaded Microspheres Delays Photoreceptor Cell Death and Vision Loss in the rd10 Mouse Model of Retinitis Pigmentosa. Invest Ophthalmol Vis Sci. 2016;57(8):3610-3618. doi:10. 1 167/iovs. 16-19300

The therapeutically effective dose of the synthetic peptide analog of the PIF peptide and of the pharmaceutical composition comprising depends on the nature of the conditions, the form of execution and the pharmaceutical formulation, and will be defined by the experience of a physician through the application of the usual investigations for a dose increase. The effective dose may be considered to be from 0.0001 to about 1000 mg/kg body weight per day, or from 0.01 to about 500 mg/kg body weight per day, or from 0.01 to 100 mg/kg body weight per day, or from 0.05 to about 10 mg/ kg body weight per day. For example, the postulated daily dose for an adult person of approximately 70 kg body weight will be in the range of 1 mg to 1000 mg, or from 5 mg to 500 mg, and can be made by single or multiple doses, daily or not.

In a particular embodiment, the pharmaceutical composition can be used for the claimed indication by administering to an individual a dose of 0.1 mg/kg/day to 1000 mg/kg/day, specifically a dose of 0.1 mg/kg/day to 500 mg/kg/day, or a dose of 1 mg/kg/day to 400 mg/kg/day, or a dose of 10 mg/kg/day to 300 mg/kg/day.

The dosage and administration interval of the pharmaceutical composition may vary depending on various factors, for example, individual conditions, such as availability and duration of the drug administration procedure, sex, age and weight of individuals, severity of diseases and the like. The specific route of administration and dosage can be selected by a person skilled in the art according to conditions such as age, weight, degree of disease activity and physical condition. The particular dosage conditions and administration interval can be easily determined by a person skilled in the art.

The invention is illustrated by the following examples which describe in detail the objects of the invention. These examples are not to be considered as limiting the scope of the invention but as illustrative thereof.

### Brief description of the figures

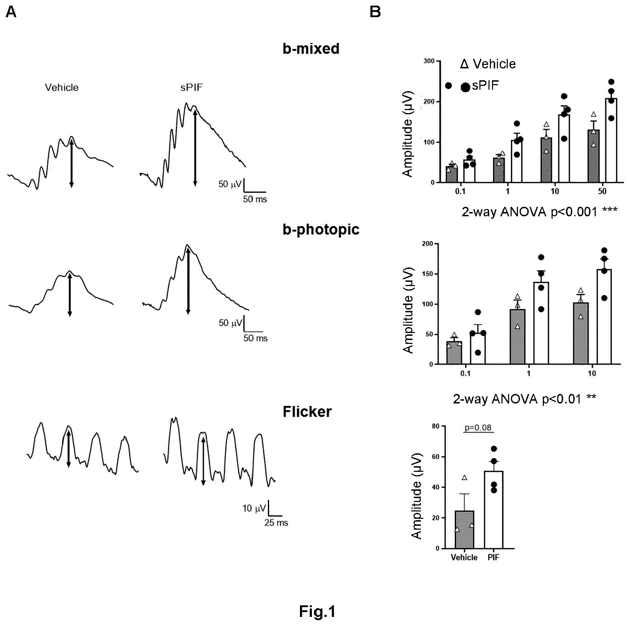

**Figure 1****. Effect of the treatment with the peptide of the invention on retinal response to light.** *rd10* mice received daily intraperitoneal injections of 50 µl of vehicle (saline) or sPIF (1 mg/kg) from P15 to P27 inclusive. Electroretinography (ERG) recordings were performed at P28. **A**. Representative ERG responses of vehicle- and sPIF-treated mice to a stimulus of 50 cd·s/m². The value of the scale bar is indicated in the figure. **B**. Graphs show mean ERG wave amplitudes, plotted as a function of light stimulus. Amplitudes of rod and cone mixed responses (b-mixed waves) to the indicated light intensities were recorded under scotopic conditions after overnight adaptation to darkness. Amplitudes of cone responses (b-photopic and flicker waves) to the indicated light intensities were recorded under photopic conditions after 5 minutes of light adaptation (30 cd·s/m²). Dots represent individual mice and bars represent the mean (+SEM) for each group. n=3-4 animals per group. ***p< 0.001; **p< 0.01 (2-way ANOVA); p=0.08 (unpaired 2-tailed Student's t test).

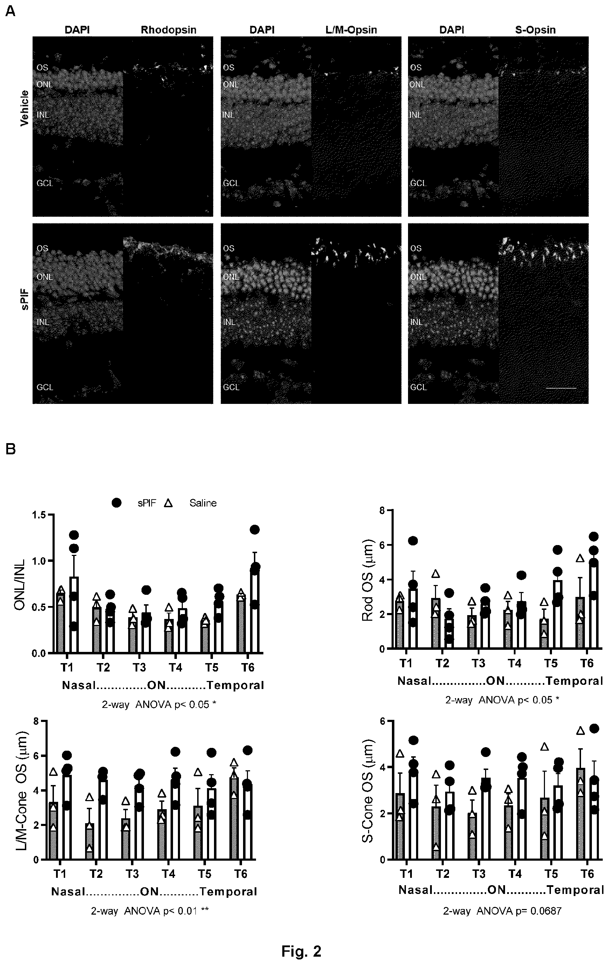

**Figure 2****. Effect of the treatment with the peptide of the invention on photoreceptor preservation**. *rd10* mice received daily intraperitoneal injections of 50 µl of vehicle (saline) or sPIF (1 mg/kg) from P15 to P27 inclusive. The retinas were analyzed at P29, after the ERG. **A.** Representative images of central retinal areas in cryosections immunostained for rhodopsin (left), L/M-opsin (centre) and S-opsin (right). Nuclei were stained with DAPI (left insets). OS, outer segments; ONL, outer nuclear layer; INL, inner nuclear layer, GCL, ganglion cell layer. Scale bar, 31 µm. **B**. ONL and INL thickness and the length of rod and cone outer segments were measured in equatorial sections corresponding to 6 regions of the retina, following a nasotemporal sequence (T1-T6) (Sanchez-Cruz, 2018). Plots show the mean + SEM. n=3-4 mice, 3 sections per retina, 3 measurements per region and section. ON, optic nerve. **p< 0.01; *p< 0.05 (2-way ANOVA).

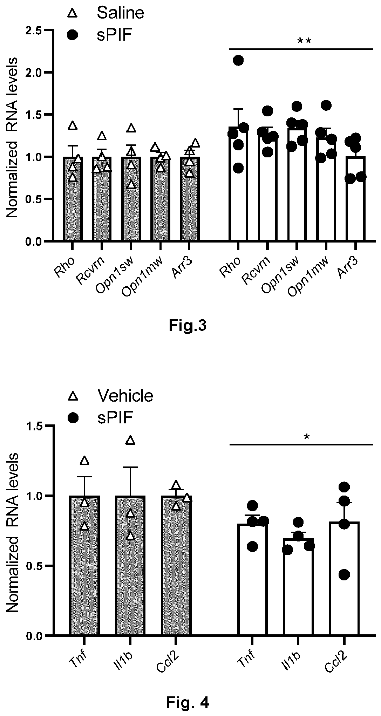

**Figure 3****. Effect of sPIF treatment on photoreceptor gene expression.** *rd10* mice received daily intraperitoneal injections of 50 µl of vehicle (saline) or PIF peptide (1 mg/kg) from P15 to P27 inclusive. RT-qPCR of individual retinas of PIF peptide- and vehicle-treated *rd10* mice at P29, after the ERG. Transcript levels were normalized to *Tbp* RNA levels and relativized to their respective WT levels (= 1). Results represent the mean + SEM. n=4 individual retinas of 4 mice. **p< 0.01 (2-way ANOVA).
**Figure 4****. Effect the treatment with the peptide of the invention on the expression of inflammatory genes.** *rd10* mice received daily intraperitoneal injections of 50 µl of vehicle (saline) or PIF peptide (1 mg/kg) from P15 to P27 inclusive. RT-qPCR of individual retinas of sPIF- and vehicle-treated *rd10* mice at P29, after the ERG. Transcript levels were normalized to *Tbp* RNA levels and relativized to their respective WT levels (= 1). Results represent the mean + SEM. n=4 individual retinas of 4 mice. *p< 0.05 (2-way ANOVA).

### Examples

### Materials and Methods

### Animals

*rd1, rd10, P23H*/*P23H,* and WT control mice were obtained from The Jackson Laboratory (Bar Harbor, ME, USA). The *rd1 (Pde6b*^{*rd1*/}*^{rd1}),* and *rd10 (Pde6b*^{*rd10*/}*^{rd10})* mice carry a recessive homozygous spontaneous nonsense and missense mutation, respectively in the rod-specific phosphodiesterase 6b gene. The *P23H*/*P23H (Rho*^{*P23H*/}*^{P23H})* mouse was generated by a knock-in strategy and carries a dominant mutation consisting of the substitution of the proline at position 23 of rhodopsin gene with a histidine. The *rd1* mouse presents, the earliest onset of the disease and most of rods are lost by postnatal day (P) 15. Both, the *rd10* and *P23H*/*P23H* models also present aggressive progression of the disease, leading to early-life blindness. In the *rd10* mouse model most of the rod photoreceptor loss occurs between P18 and P30. In the case of the *P23H*/*P23H* mouse model most of the rods die between P14 and P21. All animals were bred on a C57BL/6J background, and were housed and handled in accordance with the ARVO statement for the Use of Animals in Ophthalmic and Vision Research and the guidelines of the European Union and the local ethics committees of the CSIC and the Comunidad de Madrid. Mice were bred and housed at facilities on a 12/12-h light/dark cycle. Light intensity was maintained at 3-5 lux.

### Electroretinography (ERG) recordings

Electroretinographic responses were recorded using a standard ERG recording equipment. Many examples of those are described in the state of the art and a skilled person would know which equipment can be used. ERG signals were amplified and band filtered between 0.3 Hz and 1000 Hz using a PowerLab T15 acquisition data card (AD Instruments Ltd., Oxfordshire, UK). Mice were maintained in darkness overnight. The next day animals were anesthetized in scotopic conditions with ketamine (50 mg/kg; Ketolar, Pfizer, New York, NY, USA) and medetomidine (0.3 mg/kg; Domtor, Orion Corporation, Espoo, Finland), and their pupils dilated with a drop of tropicamide (Alcon, Fort Worth, TX, USA). Next, the ground electrode was located parallel to the tail of the animal and the reference electrode was placed into the mouth. Methocel (Colorcon, Harleysville, PA, USA) was applied to the cornea to avoid drying and the corneal electrode was placed in contact with the Methocel. ERG recordings were first obtained in scotopic conditions with increasing light stimuli (0.1, 1, 10 and 50 cd·s/m2). After light adaptation at 30-50 cd/m2 (photopic conditions), ERG response was measured at increasing light stimuli (1, 10 and 50 cd·s/m2). After ERG recording, sedation was interrupted with atipamezol (1 mg/kg; Antisedan, Orion Corporation, Espoo, Finland). All measurements were performed by an observer blind to the experimental condition. Wave amplitude was analyzed using Labchart 7.0 software (AD Instruments, Oxford, UK). The animals were euthanized the following day after the ERG and one eye was processed for histological analysis and the other for either RNA isolation and quantitative PCR or protein extraction, as described below.

### RNA isolation and quantitative PCR

Total RNA was isolated from tissues using Trizol reagent (ThermoFisher Scientific, Boston, MA, USA). Before RT, potentially contaminating DNA was eliminated with DNAse I (ThermoFisher Scientific). RT was performed with 1 µg RNA and with the Superscript III Kit and random primers (all from ThermoFisher Scientific). Quantitative (q)PCR was performed with the ABI Prism 7900HT Sequence Detection System using TaqMan Universal PCR Master Mix, no-AmpEthrase UNG, and Taqman assays (listed below) for detection (all from ThermoFisher). Relative changes in gene expression were calculated using the ΔCt method, normalizing to expression levels of the *Tbp* (TATA-binding protein) gene. The primer-probe sets used are listed in Table 1.

**Table 1. TaqMan Assays probes**

| **Gene** | **Probe** | **NCBI Reference Sequence** |
|---|---|---|
| *Arr3* (cone arrestin) | Mm00504628_m1 | NM_133205.3 |
| *Opn1mw* (L/M-Opsin) | Mm00433560_m1 | NM_008106.2 |
| *Opn1sw* (S-Opsin) | Mm00432058_m1 | NM_007538.3 |
| *Rcvrn* (Recoverin) | Mm00501325_m1 | NM_009038.2 |
| *Rho* (Rhodopsin) | Mm01184405_m1 | NM_145383.1 |
| *Tbp* (TATA-binding protein) | Mm01277042_m1 | NM_013684.3 |
| *Ccl2* (chemokine (C-C motif) ligand 2) | Mm00441242_m1 | NM_011333.3 |
| *Il1β* (Interleukin 1 beta) | Mm00434228_m1 | NM_008361.3 |
| *Tnfα* (Tumor Necrosis Factor alpha) | Mm00443258_m1 | NM_001278601.1 |

### Histological analysis of retinal sections

Animals were euthanized and their eyes were enucleated and fixed for 50 minutes in freshly prepared 4% paraformaldehyde in Sorensen's phosphate buffer (SPB) (0.1 M, pH 7.4) and then cryoprotected by incubation in increasing concentrations of sucrose (final concentration, 50% in SPB). Eyes were then embedded in Tissue-Tek OCT (Sakura Finetec, Torrance, CA, USA) and snap frozen in isopentane on dry ice. Equatorial sections (12 µm) were cut on a cryostat and mounted on Superfrost Plus slides (ThermoFisher Scientific), dried at room temperature, and stored at -20°C until the day of the assay. Before performing further analyses, slides were dried at room temperature. After rinsing in PBS and permeation with 0.2% (w/v) Triton X-100 in PBS, sections were incubated with blocking buffer [5% (v/v) normal goat serum, 1% (w/v) Tween-20, 1 M glycine in PBS] for 1 h and then incubated overnight at 4°C with primary antibodies (Table 2) diluted in blocking buffer. Sections incubated in the absence of primary antibody were used as specificity controls. After rinsing in PBS and incubation with the appropriate secondary antibodies (Table 2), sections were stained with DAPI (4',6-diamidino-2-phenylindole; Sigma-Aldrich Corp., St. Louis, MO, USA) and cover slipped with Fluoromount-G (ThermoFisher Scientific).

Sections were analyzed using a laser confocal microscope (TCS SP5 and TCS SP8; Leica Microsystems, Wetzlar, Germany). In all cases, retinal sections to be compared were stained and imaged under identical conditions.

To assess preservation of the photoreceptor layer we compared the thickness of the ONL (which primarily contains photoreceptors) with that of the corresponding INL (which contains bipolar, horizontal, and amacrine neurons and Müller glial cell bodies). ONL thickness was normalized to that of the INL, which is not affected by degeneration at this stage (Barhoum et al., 2008; Sakami et al., 2011), in order to correct for possible deviations in the sectioning plane. Two-three sections per retina were analyzed: for each section, six areas in a nasotemporal sequence were photographed (Sanchez-Cruz, 2018). ONL and INL thickness were measured in three random positions for each image. The length of rod and cone outer segments (OS) was evaluated by measuring the length rhodopsin, L/M cone and S-cone staining. In each image, 3 measurements were recorded at random positions to obtain an average value per retinal zone per section. Measurements were acquired using the "freehand line" and "measure" tools in Fiji software.

**Table 2. List of antibodies and fluorophores**

| **Antibody** | **Host species** | **Dilution** | **Manufacturer** | **Catalog number** | **UniProt Number** |
|---|---|---|---|---|---|
| L/M-opsin | Rabbit | IH, 1:200 | Millipore, Burlington, MA, USA | AB5405 | P04000 |
| Rhodopsin | Mouse | IH,1:500 | Abcam, Cambridge, UK | AB3267 | P08100 |
| S-opsin | Goat | IH,1:200 | Santa Cruz, Santa Cruz, CA, USA | SC14363 | P03999 |
| Anti-Immunoglobulins-Alexa 488-546-647 | Goat | IH, 1:200-500 | ThermoFisher Scientific, Waltham, MA | A-11001 | |
| | | | | A-11008 | |
| | | | | A-11004 | |
| | | | | A-11011 | |
| | | | | A-21235 | |
| Anti-Immunoglobulins-Alexa 568 | Donkey | IH, 1:200 | ThermoFisher Scientific | A-11057 | |

### Preimplantation Factor treatment

Synthetic Preimplantation Factor (sPIF) (MVRIKPGSANKPSDD) was synthesized in the Protein Chemistry facility in the CIB-Margarita Salas by solid-phase peptide synthesis (Peptide Synthesizer, AAPPtec, Focus XC; Louisville, KY, USA) employing Fmoc (9-fluorenylmethoxycarbonyl) chemistry. Final purification was carried out by reversed-phase HPLC and identity was verified by linear ion trap LC/MS with electrospray ionization and amino acid analysis at >95% purity.

Mice received daily intraperitoneal injections of 50 µl vehicle (saline) or sPIF (1 mg/kg) for the indicated period of time in the next section.

### Statistical analysis

Statistical analyses were performed with GraphPad Prism software 8.0 (GraphPad Software Inc., La Jolla, CA, USA). The analysis of more than two data sets was done using one-way ANOVA. In these cases, to compare the values of different experimental conditions with those of a control condition, Dunnett's multiple comparison test was used. If several comparisons between preselected conditions were carried out Sidak's post-test was employed. Comparisons of two variables were performed using a 2-way ANOVA, followed by Sidak's multiple comparison post-hoc test in cases in which a significant interaction between both variables was found. In all cases, statistical significance was established at p <0.05.

### Results

### Treatment with sPIF ameliorated RP progression.

We treated the *rd10* mice with sPIF. *rd10* littermates received daily intraperitoneal injections of either vehicle or sPIF (1 mg/kg) starting at P15 (i.e. before the appearance of evident retinal degeneration) up to P27 (after the pick of rod loss) and the visual function was assessed by ERG at P28. Compared with the vehicle-treated counterparts, sPIF-treated *rd10* mice displayed better-defined and greater amplitude ERG waves than untreated mice (Fig. 1A). Waves corresponding to both rods and cones (b-mixed waves) and cones (b-photopic waves) were of a significantly greater amplitude in sPIF-treated than in control *rd10* mice (Fig. 1B). We observed a similar trend towards increase in the Flicker amplitude, that also reflects cone response (Fig. 1B).

Histological evaluation of retinas at the end point of the study (P29, after the ERG) revealed a greater relative ONL thickness in sPIF- versus vehicle-treated retinas despite the advanced degeneration stage (Fig. 2A and B). RP courses with shortening of the photoreceptor outer segments (OS). Specific immunostaining for rod and cone OS showed better preservation of photoreceptor structure in sPIF-treated retinas (Fig. 2A). Rhodopsin immunostaining in rod OS confirmed their partial preservation in sPIF-treated retinas. By contrast, rod OS were barely present in vehicle-treated retinas. Similarly, L/M - and S-opsin immunostaining revealed longer cone OS in sPIF- versus vehicle-treated *rd10* retinas (Fig. 2A and B). In agreement with better preserved photoreceptor histology, RT-qPCR analysis revealed higher expression of photoreceptor genes in the sPIF-treated *rd10* retinas (Fig. 3). In addition, sPIF treatment decreased the expression of proinflammatory markers in the *rd10* retinas (Fig. 4).

Overall, these results demonstrate that PIF peptide has a therapeutically effect on patients suffering of RP.

### List of sequences

**SEQ_ID No 1:** synthetic peptide analog of the Preimplantation factor (PIF) peptide Xaa₁ Xaa₂ R₃ I₄ K₅ P₆ Xaa₇₋₂₀
**SEQ_ID No 2:** PIF peptide MVRIKPGSANKPSDD

## Claims

1. A synthetic peptide analog of the Preimplantation factor (PIF) peptide with a sequence length of maximum 20 amino acids consisting in the sequence:
Xaa₁Xaa₂R₃I₄K₅P₆Xaa₇₋₂₀ (SEQ ID No1): wherein:
Xaa can be any amino acid
for use in the treatment and/or prevention of Retinitis Pigmentosa.

2. The synthetic peptide according to the preceding claim, wherein the sequence length is of 10 to 20 amino acids residues, preferably of 11 to 19 amino acid residues, more preferably of 12 to 18 amino acid residues, even more preferably of 13 to 17 amino acid residues and even more preferably of 14 to 16 amino acid residues.

3. The synthetic peptide according to any one of claims 1 or 2, with an identity of at least 90%, preferably 95% with respect to the SEQ_ID No 2 for use in the treatment and/or prevention of Retinitis Pigmentosa in a patient.

4. The synthetic peptide according to the preceding claim, wherein the amino acid sequence is MVRIKPGSANKPSDD.

5. A pharmaceutical composition for use in the treatment and/or prevention of Retinitis Pigmentosa, which comprises at least one synthetic peptide analog of the Preimplantation factor (PIF) peptide as defined in any of claims 1-4, or a pharmaceutically acceptable salt of said peptide, and one or more pharmaceutically acceptable excipients.

6. The pharmaceutical composition for use according to the preceding claim, wherein the PIF peptide sequence is MVRIKPGSANKPSDD.

7. The pharmaceutical composition for use according to any of the preceding claims 5 to 6, wherein the excipients are selected from the group consisting of preservatives, agglutinants, humectants, emollients, antioxidants, water, saline buffers, and mixtures of water in oil or oil in water.

8. The pharmaceutical composition for use according to any of the claims 5 to 7, which is selected from the group consisting of solutions, creams, lotions, unguents, emulsions, and suspensions.

9. The pharmaceutical composition for use according to any of the claims 5 to 8, which is administered to a patient topically, intranasally, orally, subcutaneously, intravitreally, subconjunctivally, intramuscularly or eye drops.
